# EUROPEAN PATENT APPLICATION

(11) **EP 2 397 149 A1**
(43) Date of publication of application: **21.12.2011**
(21) Application number: 10166349.0
(22) Date of filing: 17.06.2010
(51) Int. Cl.: A61K 38/04, A61P 35/02

(54) **Pharmaceutical preparation comprising actinomycin D for the treatment of B-CLL in patients having del(17p)**

(71) Applicant: Hil-Invent Ges.M.B.H., 1190 Wien (AT)
(72) Inventor: The designation of the inventor has not yet been filed
(74) Representative: Sonn & Partner Patentanwälte

(57) **Abstract**

The present invention relates to a pharmaceutical preparation comprising actinomycin D to treat and/or prevent chronic lymphocytic leukemia (B-CLL) in patients having del(17p)and/or unmutated B-cell receptor status.

## Description

The present invention relates to a pharmaceutical preparation to treat chronic lymphocytic leukemia.

Chronic lymphocytic leukemia (B-CLL) which affects mainly elderly patients is characterized by the slow accumulation of CD5/CD19 positive B-cells in the periphery, lymph nodes and spleen. In the US and Europe 20,000 patients are newly diagnosed each year with B-CLL resulting in an overall population of 120,000 CLL patients. The course of the disease is highly variable from patient to patient making the identification and interpretation of prognostic markers an important task for clinicians and clinical scientists. Negative prognostic markers in B-CLL are high expression of the protein CD38 (> 30 %), ZAP-70 (> 20 %) on B-CLL cells, unmutated B-cell receptor as well as chromosomal aberrations. Del13q14 is present in more than half of B-CLL patients but as a single mutation does not negatively influence overall survival. Trisomy 12, del11q and del(17p) have also been reported in B-CLL in a range of 5-15 % of patients depending on the respective study. All of this 3 aberrations have been linked to shorter overall survival with del(17p) having the strongest negative impact. In patients without aberrations the mean overall survival from the time of analysis is around 69 month whereas in patients with p53 deletion or mutation it is dramatically reduced to 7.6 months (Zenz T. et al., Blood 112 (2008):3322-3329). This stresses the importance of new therapeutic options improving the outcome for this class of patients.

It is an object of the present invention to provide means to prevent and treat chronic lymphocytic leukemia.

It turned out that actinomycin D can be effectively used to prevent and/or treat chronic lymphocytic leukemia in subjects suffering from said disease and carrying del(17p) and/or unmutated B-cell receptor. Therefore the present invention relates to a pharmaceutical preparation comprising actinomycin D to treat and/or prevent chronic lymphocytic leukemia (B-CLL) in patients having del(17p) and/or unmutated B-cell receptor.

Patients having del(17p) and suffering from B-CLL have a significantly reduced life expectancy. Furthermore some of the regularly used drugs to treat B-CLL show a reduced efficacy in such patients. It turned surprisingly out that these patients can be successfully treated with a pharmaceutical composition comprising actinomycin D (Döhner et al. NEJM 343 (2000): 1910-1916).

### Besides del(17p) which is the strongest negative prognostic marker

also patients with unmutated B-cell receptor have reduced time to treatment and are thus high risk patients (Damle et al., Blood 94 (1999): 1840-1844). B-CLL cells of this subgroup respond similarly well to actinomycin D treatment.

Actinomycin D was first characterized in the 1940 and can be obtained from Strepomyces parvulus (US 2,378,876). In chemical terms it is a planar phenoxazinone ring with two pentapepeptide rings attached. It was widely used to treat different tumor types but due to toxicity its use has been cut down to a few rare tumors like Wilms tumour, Rhabdomyosarkoma, Ewing Sarcoma, Chorion Carcinoma and testicular cancer where it is still in use as a single agent but also in combination regimens with vincristine, methothrexat, chlorambucil and others.

The preparation according to the present invention is particularly efficient in treating patients suffering from B-CLL having del(17p) which are resistant to fludarabine. Fludarabine, a purine analog, is highly effective in the treatment of chronic lymphocytic leukemia. However, some patients suffering from B-CLL, in particular patients having del(17p), cannot be treated with fludarabine.

Actinomycin D is regularly used, for instance, in the assessment of the half life of RNA as an unspecific inhibitor of RNA synthesis in a concentration range of 5-10 µM (Shanmugam et al., Biochem. J. 108 (1968): 741-748). In the present invention actinomycin D is most preferably used at a much lower concentration of 0.10-0.02 µM so that apoptosis in B-CLL cells of high risk patients defined by del(17q) and/or unmutated B-cell receptor status is induced. In one study *in vitro* experiments of actinomycin D and B-CLL cells in subtoxic (0.008-0.016 µM) concentrations to sensitize B-CLL cell to death receptor TRAIL induced apoptosis are described (Olsson et al., Leukemia 15 (2001): 1868-1877). However, the goal of Olsson et al. was not to use actinomycin D as a single therapeutic agent it was the scientific question whether there are substances that can sensitize B-CLL cells, which are generally unresponsive to death receptor induced apoptosis, to TRAIL. Moreover, in contrast to the present invention patients in Olsson et al. were not classified according to their risk status, nor was it shown that actinomycin D acts specifically in different subgroups of B-CLL. In the examples provided herein actinomycin D was administered to mouse models of B-CLL (Bichi et al., Blood 99 (2002): 6955-6960) which are able to mimic the complex interactions of the B-CLL cells with their microenvironment in the *in vivo* situation. These mouse models have been successfully used to test new therapeutic options for B-CLL patients (Johnson et al., Blood 108 (2006): 1334-1338)

According to a preferred embodiment of the present invention the preparation comprises 0.1 to 5 mg, preferably 0.2 to 2 mg, more preferably 0.3 to 1 mg, in particular 0.5 mg, actinomycin D.

According to the present invention actinomycin D may be provided as a powder or as a ready-to-use solution. Such a solution may comprise actinomycin D in a concentration of 0.5 mg actinomycin D solubilized in 3 ml water. Said water may comprise at least one excipient such as mannitol in an amount of 5 to 50 mg, preferably 10 to 30 mg, in particular 20 mg.

According to a further preferred embodiment of the present invention actinomycin D is administered to a patient in an amount between 0.005 and 0.5, preferably between 0.01 and 0.2 mg, more preferably between 0.01 and 0.1, most preferably between 0.015 and 0.040 mg/kg body weight.

The preparation according to the present invention can be administered in various ways. It is particularly preferred to provide the preparation of the present invention in an oral or parenteral dosage form.

The oral dosage form is preferably a capsule, a tablet, a liquid solution, a liquid suspension or a paste.

Oral pharmaceutical dosage forms may be as a solid or liquid. Examples of solid dosage forms include, but are not limited to tablets, capsules, granules and bulk powders. More specific examples of oral tablets include compressed, chewable lozenges and tablets that may be enteric-coated, sugar-coated or film-coated. Examples of capsules include hard or soft gelatin capsules. Granules and powders may be provided in non-effervescent or effervescent forms. Each may be combined with other ingredients known to those skilled in the art.

The tablets, pills, capsules, troches and the like may optionally contain one or more of the following ingredients, or compounds of a similar nature: a binder, a diluent, a disintegrating agent, a lubricant, a glidant, a sweetening agent and a flavoring agent.

Examples of binders that may be used include, but are not limited to, microcrystalline cellulose, gum tragacanth, glucose solution, acacia mucilage, gelatin solution, sucrose, and starch paste.

Examples of lubricants that may be used include, but are not limited to, talc, starch, magnesium or calcium stearate, lyco-podium and stearic acid.

Examples of diluents that may be used include, but are not limited to, lactose, sucrose, starch, kaolin, salt, mannitol, and dicalcium phosphate.

Examples of glidants that may be used include, but are not limited to, colloidal silicon dioxide.

Examples of disintegrating agents that may be used include, but are not limited to, crosscarmellose sodium, sodium starch glycolate, alginic acid, corn starch, potato starch, bentonite, methylcellulose, agar and carboxymethylcellulose.

Examples of coloring agents that may be used include, but are not limited to, any of the approved certified water soluble FD and C dyes, mixtures thereof, and water insoluble FD and C dyes suspended on alumina hydrate.

Examples of sweetening agents that may be used include, but are not limited to, sucrose, lactose, mannitol and artificial sweetening agents such as sodium cyclamate and saccharin, and any number of spray-dried flavors.

Examples of flavoring agents that may be used include, but are not limited to, natural flavors extracted from plants such as fruits and synthetic blends of compounds that produce a pleasant sensation, such as, but not limited to peppermint and methyl salicylate.

Examples of wetting agents that may be used include, but are not limited to, propylene glycol monostearate, sorbitan monooleate, diethylene glycol monolaurate, and polyoxyethylene lauryl ether.

Examples of anti-emetic coatings that may be used include, but are not limited to, fatty acids, fats, waxes, shellac, ammoniated shellac and cellulose acetate phthalates.

Examples of film coatings that may be used include, but are not limited to, hydroxyethylcellulose, sodium carboxymethylcellulose, polyethylene glycol 4000 and cellulose acetate phthalate.

The parenteral dosage form is preferably an injection solution adapted for intradermal, intramuscular, intravenous or subcutaneous administration.

Solutions used for parenteral, intradermal, intramuscular, subcutaneous, or intravenous administration may optionally include one or more of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; agents for the adjustment of tonicity such as sodium chloride or dextrose, and agents for adjusting the acidity or alkalinity of the composition, such as alkaline or acidifying agents or buffers like carbonates, bicarbonates, phosphates, hydrochloric acid, and organic acids like acetic and citric acid. Parenteral preparations may optionally be enclosed in ampules, disposable syringes or single or multiple dose vials made of glass, plastic or other suitable material.

The preparation of the present invention may comprise next to actinomycin D other ingredients such as excipients, preservatives etc. as outlined above. Furthermore said preparation may also comprise other drugs which may support the treatment of B-CLL. Therefore the preparation of the present invention may further comprise fludarabine, cyclophosphamide, chlorambucil, bendamustine, the monoclonal antibodies rituximab (anti-CD20) and alemtuzumab (anti-CD52). All of these compounds are well known in the art and regularly used in the treatment of cancer.

Another aspect of the present invention relates to the use of actinomycin D for the preparation of a pharmaceutical preparation according to the present invention to treat and/or prevent chronic lymphocytic leukemia (B-CLL) in patients having del (17p).

A further aspect of the present invention relates to a method for treating and/or preventing chronic lymphocytic leukemia (B-CLL) in patients having del(17p) by administering to said patients a pharmaceutical preparation according to the present invention.

The present invention is further illustrated in the following figures and examples, however, without being restricted thereto.

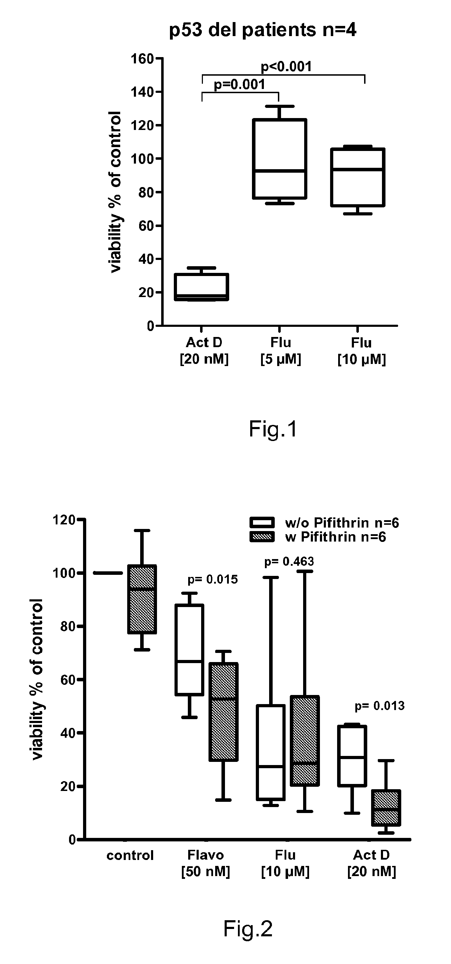

Fig. 1 shows the efficacy of actinomycin D in 4 different p53 deleted, fludarabine resistant B-CLL patients: Cells were treated with 20 nM actinomycin D for 48 h. Viability was measured using flow-cytometry (AnnexinV/7-AAD,-/-). Actinomycin D treatment resulted in a significant viability reduction in p53 deleted B-CLL cells (p < 0.001). In contrast, treatment with 5 µM or 10 µM fludarabine had no significant influence on cell viability. The length of the box corresponds to the interquartile range (IQR) and is the difference between the 75th and 25th percentiles. The horizontal line in the box is the median. The whiskers denote the lowest an the highest value measured.
Fig. 2 shows the influence of the p53 inhibitor pifithrin-alpha on flavopiridol, fludarabine and actinomycin D susceptibility. B-CLL cells from 6 different donors were treated with 50 nM flavopiridol, 10 µM fludarabine or 20 nM actinomycin D with or without 30 µM pifithrin-alpha and incubated for 48 h. Viability of B-CLL cells was assessed using a flow-cytometer (Annex-inV/7-AAD -/-). The length of the box corresponds to the interquartile range (IQR) and is the difference between the 75th and 25th percentiles. The horizontal line in the box is the median. The whiskers denote the lowest an the highest value measured.

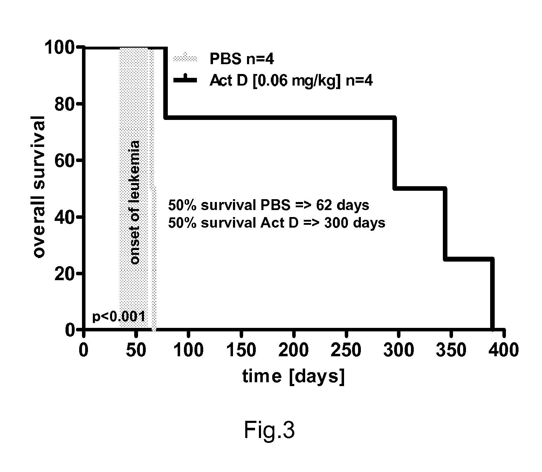

Fig. 3 shows the Kaplan-Maier curve of engrafted B-CLL mice treated with actinomycin D as compared to control mice. Day zero is the day of engraftment with 3x10⁶ lymphocytes derived from TCL1 transgenic mice with overt leukemia. As indicated by the light grey field engrafted mice develop overt leukemia at about 40 to 55 days after engraftment. Mice have obtained three treatment schedules with actinomycin D.

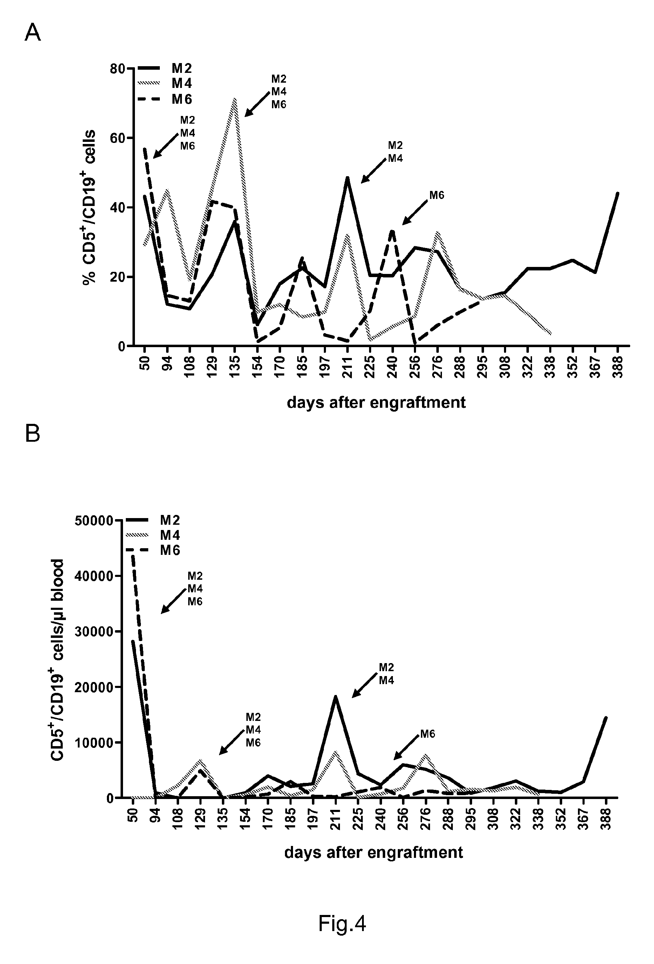

Fig. 4 shows the relative (A) and total (B) tumour load (CD5/CD19 cells) in the three actinomycin D treated mice. Blood samples were collected from the tail vein of the respective animal and whole blood was analysed for CD5/CD19 positive cells using a flow-cytometer. Arrows indicate time of actinomycin D treatment. Surviving actinomycin D treated mice were M2, M4 and M6. Mouse M6 stayed in remission considerably longer after second treatment and was therefore retreated at a later time point.

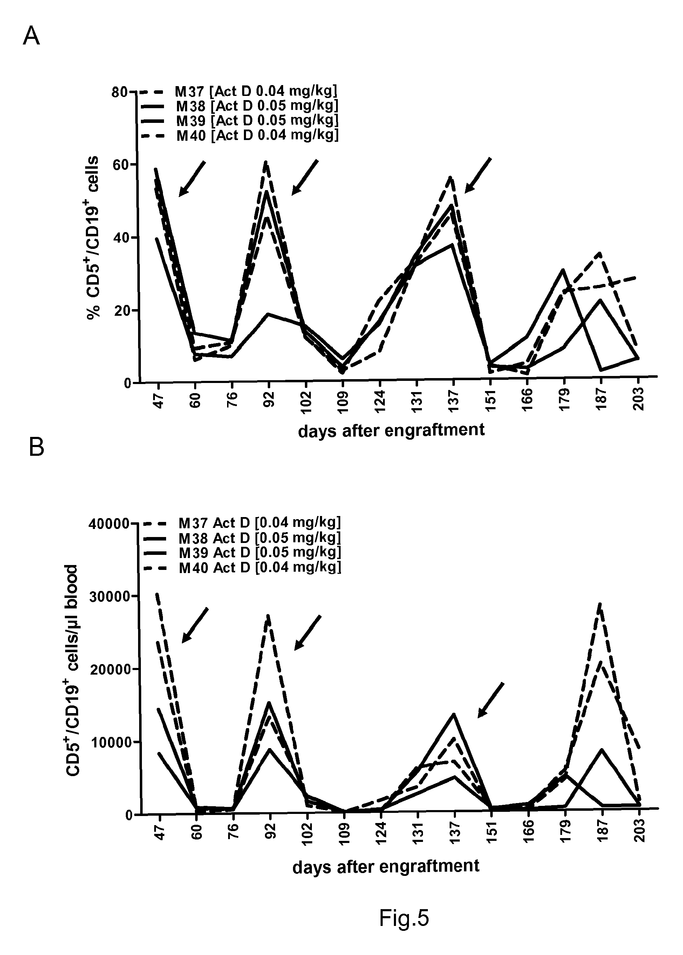

Fig. 5 shows the relative (A) and total (B) tumour load (CD5/CD19 cells) before and after treatment with reduced amounts of actinomycin D. Blood samples were collected from the tail vein of the respective animal and whole blood was analysed for CD5/CD19 positive cells using a flow-cytometer. Dashed line is 0.04 mg/kg and black line is 0.05 mg/kg daily for two weeks. Mice have now undergone two treatment schedules (red arrow)

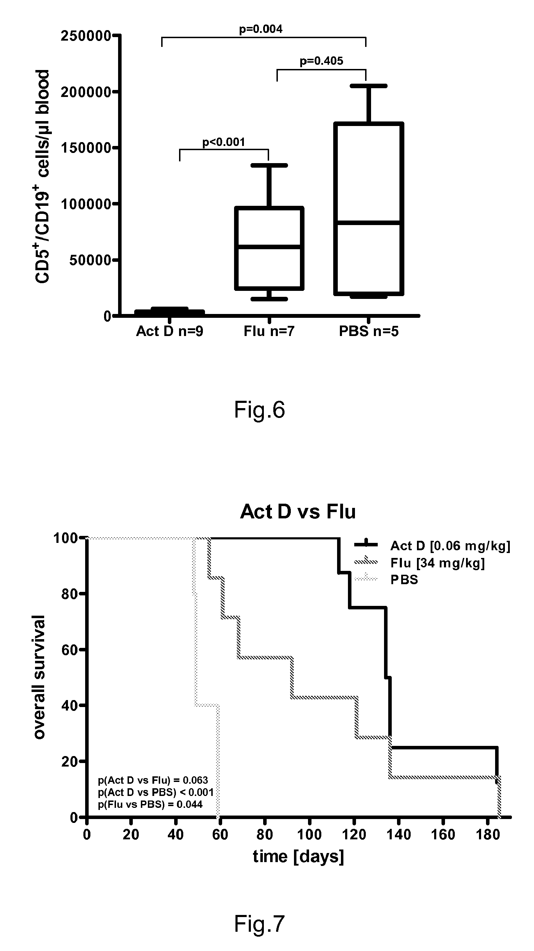

Fig. 6 shows tumour load (CD5+/CD19+ cell count) of engrafted wildtype (C57BL/6) mice after treatment with fludarabine or actinomycin D. Fludarabine is not able to reduce the tumour load but actinomycin D leads to a highly significant tumour load reduction(p = 0.004). Timepoint of this measurement was 22 days after treatment start.
Fig. 7 shows the overall survival of engrafted wildtype (C57BL/6) mice treated with fludarabine, actinomycin D or control (PBS). The mice were grouped into 3 cohorts, the actinomycin D (0.06 mg/kg by 14 days daily injections) group (n=8), the fludarabine (34 mg/kg by 5 days daily injections) group (n=7), and the control group (n=5, treated with PBS in the same injection schedule). The 50% survival from start of treatment was 103, 47 and 14 days, respectively.

### EXAMPLES:

### Materials and Methods

### Cell lines and screen substances

The chromosomal aberration del(17p)13 in B-CLL has severe prognostic and therapeutic consequences. It leads to therapy resistance and dramatically reduced overall survival. Therefore, in the initial screen we used a cell line that was p53 defective (CEM-C7H2) and derived from a patient with acute lymphoblastic anemia. This cell line carries two heterozygous mutations in the p53 gene (R175H, R248Q) (Geley S. et al., Cancer Res. 56 (1996): 5033-5038). SHP-77 (small cell lung cancer) and WS-1 (skin keratinocytes) were ordered from the American Type Culture Collection. The screen substance collection was from Analyticon Discovery (Germany). Resupply of each substance is warranted by the company. For primary B-CLL cells RPMI 1640 medium was used, supplemented with 10 % fetal calf serum, PAA Laboratories, Linz. For the cell lines (HepG2, SHP-77, HEK-293, WS-1) media recommended by the ATCC strain collection were used. Actinomycin D for mice experiments was purchased from Sigma-Aldrich (St. Louis, MO) and Cephaeline was obtained from Analyticon Discovery (Potsdam, Germany). The respective concentrations and application schedules are given in the results section.

### Substance screen in CEM-C7H2 cells

The supplied substances were solved in 100 µl medium at 37°C 90 min on a rotation shaker and transferred to a 96-well plated. Then 100µl medium containing 0.4 x 10⁶ CEM-C7H2 cells was added. The final concentration of the substances was 20 µM.

Plates were incubated 24 and 48 hours. At each time point 100 µl were removed and mixed with a XTT-dye solution and incubated for another 5 hours. As a positive control cell were put into medium without any test substance. Negative controls were 8 and 16 µM arsenic trioxide and 400 nM ABT-737 a specific Bcl2p Inhibitor. Additionally, a negative control without cells but with XTT-dye was added. Absorption was measured at 450 nm and correlated to a reference wavelength of 655 nm. Only substances that resulted in a > 80 % reduction in dye development as a readout for cell metabolism were counted as hits.

### IC50 determination

1.5 x 10⁶ B-CLL cells and 0.4 x 10⁶ CEM-C7H2 cells per well were incubated with different concentrations of the respective substance (0,2, 0,5, 1, 2, 5, 10, 20 µM) and incubated for 48 h. After this XTT-dye solution was added as described above and absorption was measured at 450 nm with 655 nm as reference wavelength. Concentrations that led to a 50 % metabolism reduction were monitored as IC50 value.

### Substance screen with CLL cells

The supplied substances were solved in 100 µl medium at 37°C 90 min on a rotation shaker and transferred to a 96-well plated. Then 100µl medium containing 1.5 x 10⁶ B-CLL cells was added. Final concentration of the substances was 20 µM. After a incubation time of 48 h 50 µl XTT-dye was added and absorption measured as above. Positive and negative controls were as above except the concentrations differed (2 and 4 µM arsenic trioxide, 100 nM ABT-737) due to the higher sensitivity of B-CLL cells. Only substances that reduced the cell metabolism/dye development > 50 % were counted. The reason for this higher cut off value is the low proliferative activity of B-CLL cells who are mainly in Go arrest. Positive substances were double checked with Annex-inV/7-AAD apoptosis/necrosis staining in CD19+/CD5+ positive cells on a flow cytometer.

### Murine Experiments

The original Eµ-TCL1a transgenic mice which were generously provided by C. Croce (Ohio) have been back-crossed for > 9 generations to C57BL/6 mice. Mice were killed at stated ages, or when signs of illness developed, and samples for histology, flow cytometry, and miRNA isolation were collected. The tumor load in all lymphoid organs was assessed by flow cytometry following staining for the surface markers CD19, CD5, and IgM. Approval from the Austrian Animal Ethics committee was obtained prior to performing the experiments. Engrafted mice were produced by injecting 3 x 10⁶ lymphocytes of a leukemic Eµ-TCL1a transgenic mouse into 30 Black 6 mice from Charles River Laboratories, Sulzfeld, Germany. These mice develop leukemia within 6-8 weeks after engraftment and the median survival time after engraftment is 12 weeks.

### Example 1:

1496 substances that were preselected for maximal diversity and membrane permeability for p53 independent apoptosis inducers in a p53 deleted T-ALL cell line CEM-C7H2 were screened. The hits from this screen were quality controlled by reordering and repetition of the experiments.

In a second test all hits from the first screen were then assayed for their ability to induce apoptosis in native B-CLL cells, especially in B-CLL cells form patients with high risk characteristics (del(17p), del (11q), CD38 > 30%, unmutated B-cell receptor status). Only substances that were able to induce apoptosis in > 80 % B-CLL cells from high risk patients were selected for further analysis.

Finally, the substances were tested for their toxicity in cell lines from different parts of the human body such as liver (HepG2), kidney (HEK-293), lung (SHP-77), skin keratinocytes (WS-1) and in human umbilical vein endothelial cells from 3 different donors. 9 substances that showed least toxicity in this cell lines but were still active apoptosis inducers in B-CLL cells were selected. For these substances the mean IC50 (48h) in 4 B-CLL cells samples from 4 different donors was determined and the substances were numbered beginning with the lowest IC50 in B-CLL cells. Substance No. 1 turned out to be actinomycin D with a mean IC50 of 80 nM in vitro.

### Actinomycin D acts independently of p53 status

At 20 µM actinomycin D was able to induce apoptosis in all three B-CLL cells samples of patients with del(17p)13. To see whether p53 independent apoptosis induction can also be achieved with lower actinomycin D concentrations B-CLL cells bearing the chromosomal aberration del(17p)13 from 4 different donors were incubated with 160 nM actinomycin D which is double the concentration that was determined as in vitro IC50 in p53 wild-type B-CLL samples.

B-CLL cells of del(17p)13 patients are often resistant to fludarabine treatment which is together with cyclophosphamide and Rituximab the mainstay of current treatment protocols for B-CLL. In accordance with this notion fludarabine resistance in vitro in all B-CLL samples bearing the del(17p)13 at concentrations that were even higher (5µM, 10µM) than the double IC50 determined for fludarabine (3µM) was found. In contrast, actinomycin D led to a > 45 % viability reduction on all del(17p)13 B-CLL samples derived from peripheral blood and a viability reduction of 38 % in one sample derived from the bone marrow, showing its activity in a p53 aberrant B-CLL background. Moreover, B-CLL cells with del(17p)13 were treated with clinically feasible concentrations of fludarabine (5µM, 10µM) and actinomycin D (20 nM). As expected, the B-CLL cells of del(17p)13 patients were resistant to fludarabine treatment whereas actinomycin D was able to induce apoptosis in > 80 % of B-CLL cells within 48 h (Fig. 1).

The p53 inhibitor pifthrin-alpha 7 has been described to inhibit the transactivation of p53 downstream targets and thereby makes it possible to mimic a p53 deletion in the treated cell lines. Cell lines including the E1a+ras transformed murine cell line C8 when treated with pifithrin-alpha have shown resistance to many p53 dependent drugs like doxorubicin, etoposide and taxol 7. It was of interest to clarify whether mimicked p53 deletion by pifithrin-alpha also induces resistance to actinomyicn D or whether actinomycin D works independent of p53 activation status. B-CLL cells of patients with no p53 aberrations were treated with 160 nM actinomycin w or w/o 30 µM pifithrin-alpha. Actinomycin D as well as flavopiridol, a substance that has been shown to be active in p53 deleted patients, were synergistic with pifithrin-alpha. This suggests that actinomycin D similar to flavopiridol shows efficacy in p53 deleted patients. With fludarabine that has been shown to be ineffective in p53 deleted patients no synergism can be observed. It is important to note that pifithrin-alpha alone had no effect on viability of CLL cell with the concentrations used (Fig. 2).

Finally, the colon cancer cell line HCT116 which is available in an isogeneic form with or without a p53 deletion was tested for its sensitivity towards actinomycin D. In this case p53 deletion had no effect on the actinomycin D susceptibility. These data prompted us to test actinomycin D in a mouse model of B-CLL.

### Experiments in murine B-CLL models- "Treatment study 01"

Most of the available mouse models for B-CLL are problematic due to complex genetics (Bc12 tg x TRAF DN; PKC alpha KR mouse) or low penetrance of the disease in the genetically modified animals (APRIL Tg; DLEU2/miR-15a/miR-16-1 KO)8. Moreover, attempts to inject and engraft human B-CLL cells into immunodeficient mice have been largely unsuccessful. The injected B-CLL cells from the human donor could be detected no longer than 4-6 weeks mainly in the murine spleen. Therefore, the well described TCL1 mouse model was selected, which over expresses the TCL1 gene using the Eµ/VH B-cell specific promoter-enhancer. TCL1a transgenic (tg) mice currently represent the best described animal model for the study of B-CLL and have been used for the validation of novel therapeutic targets, and as a tool for the preclinical assessment of anti-CLL therapies. Importantly, it has been shown that the B-cell receptors of this mouse model are unmutated thus resemble those of aggressive, treatment resistant human chronic lymphocytic leukemia. The TCL1 mice represent an ideal murine model for chronic lymphocytic leukemia patients with high risk features.

B-CLL development in these mice can be divided into two distinct phases: an asymptomatic pre-leukemic phase and the development of overt disease earliest seen at 10 months, characterized by splenomegaly, hepatomegaly, enlarged lymph nodes, and the presence of leukemic cells in all lymphoid organs. However, the asymtomatic phase can be reduced to about 1 month by engrafting TCL1 mouse derived leukemic cells in healthy wild-type mice.

Two designs were used to test the efficacy of actinomycin D in mouse models.

First a treatment study included 10 mice. The mean CD5/CD19 count of these mice was 52 % indicating full blown disease. This was also reflected in the fact that 2/10 mice had already succumbed to disease at the time of therapy start.

Based on dose finding experiments in TCL1 mice 0,06 mg/kg actinomycin D was injected daily for two weeks and control mice were treated equally with phosphate buffered saline (PBS).

All 4 control mice were dead by day 66 after engraftment shortly after mock treatment. One of the actinomycin D treated mice died on day 78 after engraftment. It is noteworthy that this mouse had the highest tumour load before treatment so one can assume that the point of no return has already been passed. However, 3 of 4 actinomycin D treated mice did not show any sign of toxicity. Two of the three surviving mice in the actinomycin D arm died 296 and 344 days after engraftment without any signs of overt disease. It is remarkable that these two mice did not die of the engrafted tumour thus can be considered as healed after 3 treatment cycles. The last mouse died at day 389 after engraftment showing signs of overt disease. The difference to the mock treated animals is highly significant (Fig. 3). To assess tumour load development after treatment blood samples were collected from the tail vein (Fig. 4).

A reduction of the CD5/CD19 count can be observed in all three animals after treatment at day 50. At day 108 CD5/CD19 counts start to increase again and at day 135 reach the initial value. At that time point which is 85 days after the initial therapy the animals were retreated with actinomycin D to follow potential resistance development. Again all mice responded well to actinomycin D as reflected in the severe decrease in the CD5/CD19 count. Also at the third treatment cycle mice responded well to actinomycin D treatment suggesting no development of resistance. It has been reported that fludarabine treated animals develop resistance after the second treatment cycle mimicking the situation in B-CLL patients (Johnson AJ et al., Blood 108 (2006): 1334-1338).

### Results in correlation to other studies

In an comparable experiment using fludarabine 34 mg/kg daily for 7 days in the same mouse model it was shown that already 40 days - as opposed to 85 days in our experiments - after the initial therapy retreatment was necessary (Johnson AJ et al., Blood 108 (2006): 1334-1338). Moreover, in an attempt to test Rapamycin as an treatment option for B-CLL a slight increase in overall survival in the treated mice was observed however by day 77 after engraftment - as opposed to more than 300 days in our study - 7/8 mice already succumbed to disease (Zanesi N. et al., Cancer Res. 66 (2006): 915-920).

### Example 2:

### "Treatment study 02"- Test of reduced actinomycin D concentrations in murine B-CLL models

In example 1 a treatment schedule of 0.06 mg/kg daily for two weeks was used. The goal of the second experiment was twofold: On one hand to test the reproducibility in a newly engrafted set of CLL mice and on the other hand to test the efficacy of lower concentrations of actinomycin D. Therefore in this experiment two mice were treated with 0.05 mg/kg daily for two weeks and two mice treated with 0.04 mg/kg daily for two weeks. To assess the tumour load blood samples were consecutively taken from the tail vein. In this case 47 days after engraftment treatment was started (CD5/CD19 counts were 40 -60 %). In all mice a severe reduction of tumour cells as in our first experiment was observed. However in this case the tumour load increased to the initial values 46 days after the first treatment making a retreatment necessary. Again the tumour responded well to actinomycin D. Again 44 days later the CD5/CD19 counts increased again and a third treatment schedule was again successful in reducing tumour cells. It is important to note that after three rounds of actinmycin D also in this experiment no resistance development could be observed (Fig. 5).

### "Treatment study 03"- Head to head comparison of actinomycin D and fludarabine

The Tcl1-mouse model has been characterized as a meaningful tool for pre-clinical drug development. In this study fludarabine has been used in a schedule of 34 mg/kg for 5 days each month. The goal of this experiment was the direct comparison of actinomycin D (0.06 mg/kg daily for 14 days, 10 mice) with fludarabine (34 mg/kg for 5 days, 7 mice) and a control group (6 mice). In total 23 animals injected with lymphocytes from the same donor mouse with a mean of 40% CD5/CD19 cells of lymphocytes in the periphery were included. Thus animals which had already developed clear leukemia. One mouse from the control group and one mouse from the actinomycin D group died from injection injury during the first cycle, so 21 mice remained for the study. The tumour load reduction after actinomycin D treatment was not as severe as in treatment study 01 and treatment study 02 suggesting a comparatively more aggressive from of CLL. When the median time from engraftment to death in the treatment study 01 to the treatment study 03 67 versus 48 days, respectively, was observed again arguing for a more aggressive tumour in the treatment study 03. This gives the opportunity to compare fludarabine versus actinomycin D in a model of aggressive CLL. At day 22 after treatment start tumour load was assessed compared to that of the tumour load of untreated mice. Fludarabine reached a borderline significant tumour load reduction of 20 % (p = 0.045) whereas actinomycin D reduced the tumourload more than 80 % (p = 0.004, Fig. 6). This is reflected in the 50 % survival time after start of treatment which was 103, 47 and 14 days for the actinomycin D group, the fludarabine group and control group, respectively (Fig. 7) suggesting superiority of actinomycin D over fludarabine in aggressive B-CLL.

## Claims

1. Pharmaceutical preparation comprising actinomycin D to treat and/or prevent chronic lymphocytic leukemia (B-CLL) in patients having del(17p) and/or unmutated B-cell receptor status.

2. Preparation according to claim 1, **characterised in that** the patients having del(17p) are resistant to fludarabine.

3. Preparation according to claim 1 or 2, **characterised in that** the preparation comprises 0.1 to 5 mg, preferably 0.2 to 2 mg, more preferably 0.3 to 1 mg, in particular 0.5 mg, actinomycin D.

4. Preparation according to any one of claims 1 to 3, **characterised in that** actinomycin D is administered to a patient in an amount between 0.005 and 0.5, preferably between 0.01 and 0.2 mg, more preferably between 0.01 and 0.1, most preferably between 0.015 and 0.040 mg/kg body weight.

5. Preparation according to any one of claims 1 to 4, **characterised in that** the preparation is provided in an oral or parenteral dosage form.

6. Preparation according to claim 5, **characterised in that** the oral dosage form is a capsule, a tablet, a liquid solution, a liquid suspension or a paste.

7. Preparation according to claim 5, **characterised in that** the parenteral dosage form is an injection solution adapted for intradermal, intramuscular, intravenous or subcutaneous administration.

8. Preparation according to any one of claims 1 to 7, **characterised in that** the preparation comprises further fludarabine, cyclophosphamide, chlorambucil, bendamustine, the monoclonal antibodies rituximab (anti CD20) and alemtuzumab (anti CD52).

9. Use of actinomycin D for the preparation of a pharmaceutical preparation according to any one of claims 1 to 8 to treat and/or prevent chronic lymphocytic leukemia (B-CLL) in patients having del(17p).

10. Use according to claim 9, **characterised in that** the patients having del(17p) are resistant to fludarabine.

11. Use according to claim 9 or 10, **characterised in that** actinomycin D is administered to the patient in an amount between 0.005 and 0.5, preferably between 0.01 and 0.2 mg, more preferably between 0.01 and 0.1, most preferably between 0.015 and 0.040 mg/kg body weight.

12. Method for treating and/or preventing chronic lymphocytic leukemia (B-CLL) in patients having del(17p) by administering to said patients a pharmaceutical preparation according to any one of claims 1 to 8.

13. Method according to claim 12, **characterised in that** the patients having del(17p) are resistant to fludarabine.

14. Method according to claim 12 or 13, **characterised in that** actinomycin D is administered to the patient in an amount of between 0.005 and 0.5, preferably between 0.01 and 0.2 mg, more preferably between 0.01 and 0.1, most preferably between 0.015 and 0.040 mg/kg body weight.
